# EUROPEAN PATENT APPLICATION

(11) **EP 4 764 498 A1**
(43) Date of publication of application: **24.06.2026**
(21) Application number: 24307218.8
(22) Date of filing: 20.12.2024
(51) Int. Cl.: G01N 33/574, G01N 33/68

(54) **AN EX VIVO METHOD FOR DETERMINING A REFERENCE PROTEOMIC PROFILE OF EXTRAHEPATIC CHOLANGIOCARCINOMA IN THE PRESENCE OF BILE DUCT STRICTURE IN A SUBJECT, AND USES THEREOF**

(71) Applicant: Université de Bordeaux, 33000 Bordeaux (FR); Institut National de la Santé et de la Recherche Médicale, 75013 Paris (FR); Centre Hospitalier Universitaire de Bordeaux, 33400 Talence (FR)
(72) Inventor: SALTEL, Frédéric, 33400 Talence (FR); MARICHEZ, Arthur, 33000 Bordeaux (FR); RAYMOND, Anne-Aurélie, 33700 Merignac (FR); CHICHE, Laurence, 33600 Pessac (FR)
(74) Representative: Novagraaf Technologies

(57) **Abstract**

The present invention relates of an *ex vivo* method for determining a reference proteomic profile of extrahepatic cholangiocarcinoma in the presence of bile duct stricture in a subject, comprising steps of :
a) quantification of the protein abundances of all proteins identified in at least one bile duct cytological sample from at least one subject for which a diagnosis of extrahepatic cholangiocarcinoma has been previously established, or having benign stricture,
b) quantification of the protein abundances of all proteins identified in human bile duct cells standard reference,
c) determining the proteomic profile of each of said at least one sample by comparing protein abundances of each protein in the at least one sample with respect to the standard reference,
d) determining a reference proteomic profile for extrahepatic cholangiocarcinoma condition, by means of a statistical test established with the proteomic profiles determined in step c).

The present invention also relates to a reference proteomic profile of extrahepatic cholangiocarcinoma in the presence of bile duct stricture, and to an *ex vivo* method for differential diagnosis of extrahepatic cholangiocarcinoma in the presence of bile duct stricture of a subject.

## Description

### Technical field

The present invention refers to an ex *vivo* method for determining a reference proteomic profile of extrahepatic cholangiocarcinoma in the presence of bile duct stricture in a subject, especially for differential diagnosis of a condition in a subject.

Therefore, the present invention has utility in medical field, and more particularly in diagnosis field.

In the description below, the references into brackets ([ ]) refer to the listing of references situated at the end of the text.

### Background of the Invention

The extra-hepatic cholangiocarcinoma (eCCA) is a primitive tumor of the bile duct revealed by symptoms resulting of a bile duct stricture. Classification distinguishes distal cholangiocarcinoma (dCCA) and peri-hilar cholangiocarcinoma (pCCA) according to the anatomical location. The eCCA is diagnosed in 70% cases at non-resectable stage mostly explained by the late apparency of the symptoms. Furthermore, the bile duct stricture could be difficult to diagnose. Indeed, the actual gold-standard diagnostic test is the histological examination on cytological samples as biopsy or brushing of the bile duct obtained by endoscopic retrograde cholangiopancreatography (ERCP) or radiological interventional procedure. Sensitivity of these exams is estimated around 50%. Difficulties to make the diagnosis can lead to repetition of invasive exams having their own morbidity, indeed 5% of the ERCP are followed by pancreatitis having themselves 5% of mortality. Furthermore, negative results in biopsy can be associated to residual uncertainty leading to major surgeries for finally benign stricture. It has been shown than 17% of major hepatectomy for hilar stricture could be done for benign stricture at final exam and 5 to 10% of pancreaticoduodenectomy could also be done for benign lesions. Finally, the complexity of the interpretation of biopsies results in delay in the initiation of the therapeutics.

To overcome these issues, new diagnostics approaches have been developed. One of these is the Next-Generation Sequencing (NGS) on bile duct cytological sample which could reach 73% sensitivity alone (Singhi et al. ([1])) by searching targeted mutations in the biliary cells. It is actually recommended in the French Guidelines to perform NGS in second intention if pathological examination result is negative or indeterminate with inflammatory cells without clear diagnostic. However, 25% of false negative were reported meaning that sensitivity still must be improved.

One limitation of the biopsy and brushing is that they obtained small samples of the tumor and the bile duct with few biliary cells embedded in an abundant stroma reducing the probability of making a diagnosis with conventional histology.

Thus, a need exists of new tools for diagnosis of extrahepatic cholangiocarcinoma in the presence of bile duct stricture. The present invention fulfills these and other needs.

### Description of the invention

After intensive research, the applicants have developed an inventive approach, at another molecular level, allowing to decrease the rate of false negative by considering all cells with its microenvironment: a proteomic approach on cytological samples.

The applicants have developed a benign/malignant differential diagnosis of eCCA on cytological samples of bile duct using the profile matching proteome technique, creating a new diagnosis tool for extra-hepatic cholangiocarcinoma. This proteomic profile may be determined especially by calculating the relative abundance ratio of proteins identified in a sample compared with a cell standard. Together, these abundance ratios constitute the individual proteomic profile of each patient. A reference proteomic database is built up from typical benign or malignant cases. Proteomic profile matching consists of comparing the proteomic profile of a patient to be diagnosed with the profiles of the reference database, and evaluating the similarities.

The applicants have shown that proteomic profiles distinguished benign/malignant groups, through hierarchical clustering and principal component analysis. Extracellular and cell surface proteins were mainly involved in the signature associated with key biological functions like cell adhesion and epithelial-mesenchymal transition. Furthermore, machine learning algorithms and statistical tests achieved perfect matching for the validation cohort.

Advantageously, by accurately differentiating between benign and malignant lesions, this signature could significantly enhance clinical decision-making, reduce unnecessary surgical interventions, and improve patient outcomes.

Accordingly, in a first aspect, the present invention provides an ex *vivo* method for determining a reference proteomic profile of extrahepatic cholangiocarcinoma in the presence of bile duct stricture in a subject, comprising steps of :
a) quantification of the protein abundances of all proteins identified in at least one bile duct cytological sample from at least one subject for which a diagnosis of extrahepatic cholangiocarcinoma has been previously established, or having benign stricture,
b) quantification of the protein abundances of all proteins identified in human bile duct cells standard reference,
c) determining the proteomic profile of each of said at least one sample by comparing protein abundances of each protein in the at least one sample with respect to the standard reference,
d) determining a reference proteomic profile for extrahepatic cholangiocarcinoma condition, by means of a statistical test established with the proteomic profiles determined in step c).

"Reference proteomic profile" refers herein to a profile of protein expression established for the diagnosed pathological condition or for the benign stricture condition, which can be used later as a comparative reference for analyzing non-diagnosed tissues.

As used herein, a "subject" may be animal, mammal, or human.

The method of the invention may be realized on any cytological sample, i.e. sample composed of individual cells or small clusters of cells, collected from tissues or body fluids, that may be analyzable by mass spectrometry. It may be obtained by any method known in the state of the art, for example by at least one technique selected among microdissection, laser microdissection of a formalin-fixed paraffin-embedded or frozen tissue, fresh biopsy, and brushing. The steps of preparation of histological sections, protein extraction, preparation of samples for mass spectrometry analysis, and laser microdissection cutting, and formalin fixation reversal if any, may be realized according to methods known in the art, for example as described by Dourthe et al. ([2]) et Di Tommaso et al. ([3]).

In the method for determining a reference proteomic profile of a pathological condition or of a benign stricture condition, the sample may be obtained from at least one subject. Advantageously, the number of subjects is statistically representative. For example, the sample may be obtained from at least 5, or at least 10, or at least 15, or at least 20, or at least 30, or at least 40, or at least 50, or at least 60, or at least 70, or at least 100, or at least 150, or at least 200, or more subjects.

"Quantification" refers herein to a method allowing to extract and compare the relative abundance of identified proteins in at least two tissue samples, possibly with or without using a stable isotope containing compound labelling the proteins by mass spectrometry. Preferably, the method is a targeted quantification, preferably by mass spectrometry. The method may be any method known in the state of the art, for example as described by Sala M, et al. ([2]), Henriet E, et al. ([3]). For example, the quantitative data may be analyzed using raw processing with Thermo Proteome Discoverer software and post-processing of ratio data with R, or any other software suitable for proteomics raw data processing and post-processing. As commonly realized in this technical field, replicates may be realized for each subject, regarding pathological tissue and the standard reference. Quantification may be a label free quantification, a targeted quantification, or isotopic labelling. Targeted quantification technique may be for example a method of liquid chromatography coupled with tandem mass spectrometry (LC-MS/MS), in discovery mode in Data Dependent Acquisition (DDA) or Data-independent Acquisition (DIA), or with a targeted approach (eg. Multiple Reaction Monitoring (MRM), Parallel Reaction Monitoring (PRM)). Isobaric labelling may be for example TMT (Tandem Mass Tag, ThermoFisher) system.

"Human bile duct cells standard reference" or "human cholangiocytes standard reference" refers herein to a pathological, i.e. having extrahepatic cholangiocarcinoma, or to non-pathological, biological sample used as a benchmark or standard. The standard reference may be for example a cell line, such as immortalized human bile duct cell lines, such as H69 cells (ATCC HCM-CSHL-0433-C24), or a primary culture of bile duct cells directly derived from healthy human donors. Therefore, it may be determined a pathological human bile duct cells standard reference, and/or a non-pathological, or "normal", human bile duct cells standard reference.

Comparing protein abundances of each protein in step c) may be realized by any known method, preferably using statistical tools allowing to statistically compare protein abundances or ratios between two groups of patients or samples, such as a Wilcoxon-Mann-Whitney U-test or a t-test, this list not being limitative. The skilled person is able to choose one or more of statistical analysis regarding his knowledge of this technical field.

Advantageously, the method of the invention is a computer-implemented method, which involves the use of tools commonly used in this technical field, as for example, a record component, notably for recording, for each sample, the proteomic profile in relation to the previously established pathological condition diagnosis, a storage component for storing data, a computer processor for processing data, wherein the computer processor is coupled to the storage component and configured to execute the instructions stored in the storage component in order to receive data and analyze them according to one or more algorithms; and a display component for displaying information regarding the reference proteomic profile.

Another object of the invention relates to a reference proteomic profile, which is a "signature" of extrahepatic cholangiocarcinoma in the presence of bile duct stricture, obtained by the ex *vivo* method for determining a reference proteomic profile of extrahepatic cholangiocarcinoma in the presence of bile duct stricture in a subject as defined above.

The reference proteomic profile defines a proteomic "signature" for diagnosis of extrahepatic cholangiocarcinoma. It may comprise extracellular proteins, cell adhesion proteins and epithelial-mesenchymal transition proteins. Preferably:
- the extracellular proteins may comprise at least one protein selected in the group comprising COL15A1 (Collagen Type XV Alpha 1 Chain), MUC5B (mucin 5B), TXNDC17 (Thioredoxin Domain Containing 17), SRSF3 (Serine And Arginine Rich Splicing Factor 3), SNX1 (Sorting nexin-1), RBM12 (RNA Binding Motif Protein 12) and FKBP1A (FKBP Prolyl Isomerase 1A) protein,
- said cell adhesion proteins comprise at least one protein selected in the group comprising ITGAV (Integrin alpha-V), THBS1 (Thrombospondin-1), UTRN (Utrophin), FN1 (Fibronectin 1), ITGA5 (Integrin Subunit Alpha 5), ILK (Integrin-linked kinase), FERMT3 (Fermitin Family Member 3), VASP (Vasodilator-stimulated phosphoprotein), CFL1 (Cofilin 1), ILK, TPM4 (Tropomyosin 4), LRP1 (LDL Receptor Related Protein 1), CNN3 (calponin 3), RPS14 (Ribosomal Protein S14), FHL2 (Four And A Half LIM Domains 2) and PARK7 (Parkinsonism Associated Deglycase) protein, and
- said epithelial-mesenchymal transition proteins comprise at least one protein selected in the group comprising ITGAV, THBS1, FN1, ITGA5, LRP1 and TPM (Tropomyosin) protein.

All the definitions, explanations and embodiments given above apply mutatis mutandis to all the objects of the invention.

Another object of the invention relates to an *ex vivo* method for determining proteomic profile of a bile duct cytological sample of a subject, comprising the following steps:
- quantification of the protein abundances of all proteins identified in said bile duct cytological sample,
- quantification of the protein abundances of all proteins identified in a human bile duct cells standard reference,
- determining the proteomic profile of said at least one sample by comparing protein abundances of each protein in the at least one sample with respect to the standard reference.

Another object of the invention refers to an *ex vivo* method for differential diagnosis of extrahepatic cholangiocarcinoma in the presence of bile duct stricture of a subject, comprising the following steps:
- determining the proteomic profile of a bile duct cytological sample of the subject with a method as defined above,
- comparing the proteomic profile of said sample with a reference proteomic profile as defined above,

Wherein:
- if the proteomic profile of said sample matches with a malignant reference proteomic profile, the subject is defined as having a extrahepatic cholangiocarcinoma,
- if the proteomic profile of said sample matches with a non-cancerous proteomic profile, the subject is defined as having a benign lesion.

"Match" refers herein that the protein abundance profiles statistically correspond. For example, "match" may be defined using distance calculation (chi2 or Euclidean distance for example) and/or similarity calculation tools, such as machine learning (for example Random-Forest or Support Vector Machine).

This invention is further illustrated by the following examples with regard to the annexed drawings that should not be construed as limiting.

### Brief description of the figures

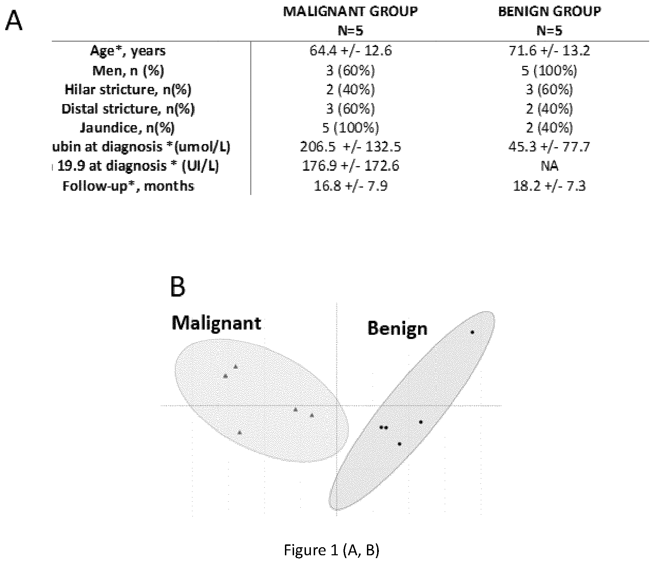

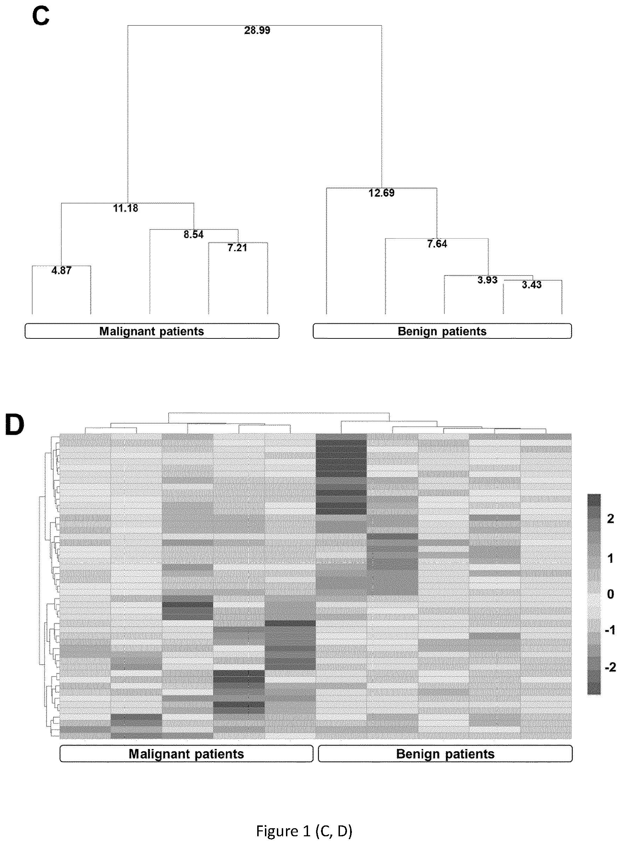

- Figure 1: represents identification of significantly deregulated proteins between benign and malignant cytological samples establishing a diagnostic proteomic signature. (A) General characteristic of the selected patients (* *Data are given in mean with standard deviation).* (B) Principal component analysis showing a clear distinction between benign and malignant cytological samples. (C) Hierarchical clustering representing a clear distinction between benign and malignant samples. (D) Heatmap of the signature showing clear separation between proteins highly expressed in malignant patients and those expressed in benign patient. (E) Boxplot showing the differential expression of the matrix proteins collagene 15A1 and fibronectin 1 between malignant and benign samples.

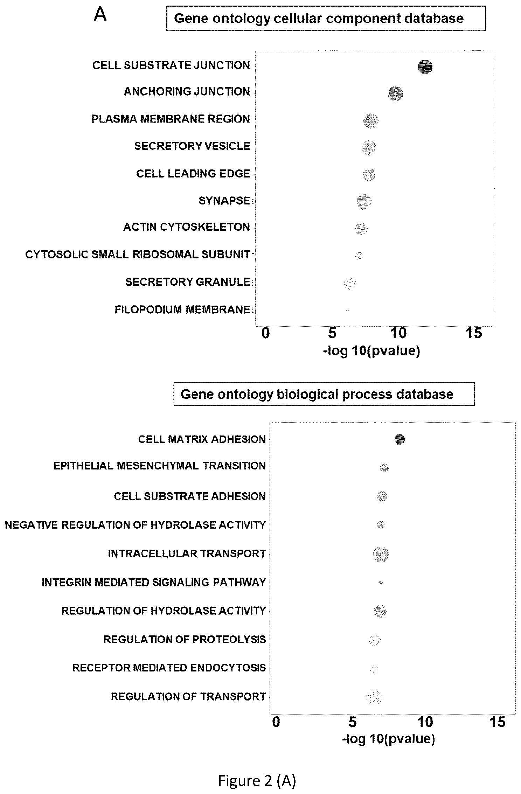

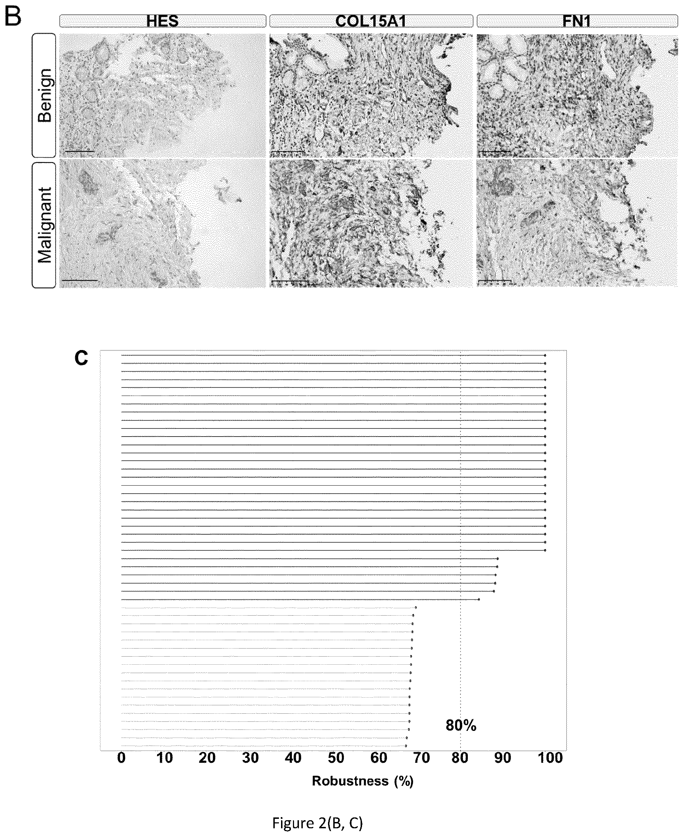

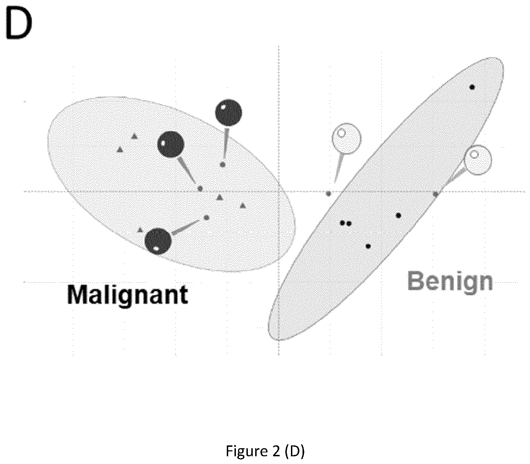

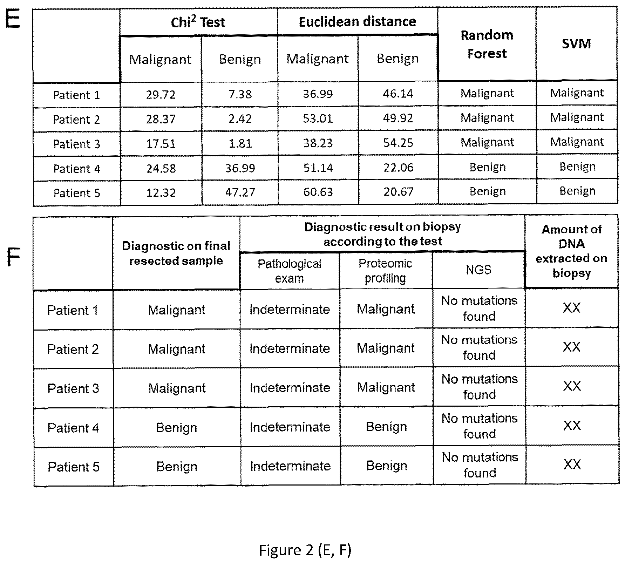

- Figure 2: represents the validation of the diagnostic signature after intern resampling and biological integrative analysis associated to a complementary analysis by next-generation sequencing (NGS). (A) Gene Set-Enrichment Analysis of the 49 proteins significantly deregulated between benign and malignant patients performed against the gene ontology cellular component database and biological process database. (B) Immunohistochemistry of a benign cytological sample and a malignant one with H&E saffron, fibronectin 1 and collagen 15A1 staining showing that they have not enough visual specificity to be used in clinical practice. (C) Bootstrap of the signature showing than 63.3% of the proteins having more 80% robustness. (D) Principal Component Analysis followed by the validation with indeterminate samples which were correctly matched with the suspected diagnosis for all of them. (E) Results from the 4 mathematical tests used (Chi2 test, Euclidean distance, Random Forest, SVM) wrote in bold when tests' results gave good matching with the suspected diagnosis. At least 3 positive tests were advised to assume that the diagnosis was correctly made. (F) NGS.

### Examples

### Example 1: Diagnosis of extrahepatic cholangiocarcinoma in the presence of bile duct stricture using proteomic profiling

### Materials and Methods

### Patients selection

From the cohort of patients having bile duct cytological samples following ERCP or interventional radiological procedures in the Bordeaux University Hospital, we selected 10 patients having a formalin-fixed paraffin-embedded (FFPE) histological blocks from biopsy and/or brushing for bile duct stricture, 5 with a diagnostic of eCCA and 5 with normal samples. All of these patients with benign results have at least one year of follow-up attesting the benignity with spontaneous regression or absence of clinical or morphological progression of the stricture.

For the validation cohort we selected 5 patients having a cytological sample for bile duct stricture and clinical suspicion of malignant lesion. All of these samples did not have any confirmed malignant cell and all of these patients had a surgery following the biopsy due to the clinical suspicion of cancer. All of them had at least 1 year of follow-up to assess their final bile duct stricture diagnosis.

All the selected samples were proofreaded by an expert anatomopathologist (GB, BLB). Our local ethic committee gives its approbation for this study, reference: CER-BDX-AP-2023-14. According to French Law, patients selected were contacted and they had the legal time to express their opposition.

### Proteomic analysis

Following the previously described methodology (Dourthe C et al. ([4])), proteomic profiling was performed on FFPE tissue as detailed below.

Microdissection was made following the area of interest selected on hematoxylin-eosin images, on a surface of 1 mm² on a 5µm-thick section with a constant stromal portion of 20 to 30% in order to obtain all the biliary cells. With the bile duct biopsies, quantity of cells was frequently poor and by definition with indeterminate strictures. We used an extract of an SV40-transformed (i.e. immortalized) normal human *cholangiocyte cell line,* H69, to establish relative protein abundance ratios (Grubman et al. ([5])). Biopsy vs. H69 ratios were compared between benign and malignant samples to establish the signature and also for the validation step. The use of the H69 cell line provides a stable, reusable source of comparison between each biopsy for each proteomic analysis. On the one hand, this guarantees the possibility of increasing the development collection over time; on the other, it is compatible with technology transfer to routine clinical use.

### Statistics and bioinformatics analysis for proteomic data

The means of protein abundance or ratio between benign and malignant samples were compared using the Wilcoxon-Mann-Whitney U-test. A p-value < 0.05 was considered statistically significant. The R version 4.3.3 was used to perform principal component analysis (PCA) with R FactorMineR and factoextra packages. Hierarchical clustering was performed using stats package. Missing data were imputed using missForest package. To evaluate the test set, we used Support Vector Machine and Random Forest algorithms (package caret), and the euclidean distance (package stats).

### Integrative biology

Gene set enrichment analysis (GSEA) was performed against Gene Ontology Database Cellular Component and Biological Process Database with Hallmarks.

### Cell culture

The cell line H69 was provided by Dr. M Avila, from the Center for Applied Medical Research of the University of Navarra, Pampelona, Spain. It was harvested with Dulbecco's Modified Eagle Medium (Gibco^{®}) F12 with 10% of decomplemented foetal bovin serum (Sigma-Aldrich) and also penicillin/streptomycin.

### Results

General characteristics of the 10 initial selected patients were reported in Figure 1A. All patients had endoscopic retrograde cholangiopancreatography (ERCP). In patients having malignant cells confirming a cholangiocarcinoma, 4 of them had surgical resection after the sample whereas none of the patients with benign biopsies had surgery. In total, 5 samples were analyzed for hilar stricture and the 5 others concerned distal stricture of the bile duct.

With systematic microdissected area of 1 mm², we identified 2676 proteins in mean with at least two unique peptides. Among them, 49 proteins were expressed significantly differently between the two groups of patients. This data set was able to distinguish benign patients and malignant patients using hierarchical clustering and principal component analysis (Fig. 1B). As illustrated by the heat map (Fig1.C), no single specific biomarker emerged from this analysis but the proteomic profiles were distinct between the two groups.

We then analyzed these 49 significant proteins in more detail, both in terms of their subcellular localization and their biological function, using a GSEA and the gene ontology cellular component database (Fig 2A). Twenty-one proteins (43%) were surface proteins of the plasma membrane, as shown by the most significant enrichment of junction and anchoring proteins (Fig 2.A). Consistently, the most significant associated biological functions were extracellular matrix adhesion, epithelial-mesenchymal transition markers and cell adhesion (Fig 2.A).

Then, we tested the most discriminating proteins by IHC to check whether they could constitute a subset of diagnostic biomarkers. The two most expressed proteins were matrix proteins, collagen 15A1 and fibronectin 1, having a ratio between malignant and benign samples of 3.78 fold and 2.81 fold respectively. The IHC labelling obtained did not reveal a sufficient differential to be used as markers (Fig.2B).

We then decided to evaluate the value of this set of 49 differentially expressed proteins between the two groups as a diagnostic signature. We first used a mathematical method of intern resampling or bootstrap to assess its stability. Among the 49 proteins, 31 proteins (63.3%) had a robustness ≥ 80% (Fig.2C). This first analysis showed good performance for this diagnostic proteomic signature.

The next step was therefore to validate it by a new set of patients. That's why we selected 5 patients having biopsies for bile duct stenosis with pathological analysis reporting indeterminate diagnosis. History of the patients is reported in table 1. All cases were analyzed with the same method.

**Table 1. Descriptive characteristics of the patients having indeterminate biopsy**

| **INDETE RMINAT E CASES** | **CLINICAL CONTEXT** | **DIAGNOSIS ON BIOPSY** | **SURGERY PERFORMED** | **FINAL DIAGNOSIS** |
|---|---|---|---|---|
| **Case 1** | Jaundice treated with repeted prothesis with several biopsies with and without cholangioscopie | Anomalies too small to be significant | Major hepatectomy with biliary reconstruction | Peri-hilar cholangiocarci noma |
| **Case 2** | Jaundice | Dystrophic cells without obivous cells suspected of malignancy | Major hepatectomy with biliary reconstruction | Peri-hilar cholangiocarci noma |
| **Case 3** | Jaundice | Extremely inflammatory wall without obvious malignant cells | Pancreaticodu odenectomy | Distal cholangiocarci noma |
| **Case 4** | Jaundice with high clinical suspicion of tumor | Absence of tumor cells | Pancreaticodu odenectomy | Benign stenosis with fibro-inflammatory remodelling |
| **Case 5** | Jaundice | Inflammatory wall without obvious malignant cells | Resection of the main bile duct with biliary reconstruction | Benign stenosis with xantogranulom atous cholecystitis |

First of all, we calculated the distance of proteomic profile of indeterminate patients with the proteomic signature (Chi2 tests and Euclidean distance). Moreover, we used two machine learning algorithms (random forest and SVM) to assign each tested case to a diagnostic group. Following these 4 tests, we obtained correct proteomic profiling matching for all the five indeterminate patients, regarding the final pathological examination (Table 1) (Fig.2E).

### Discussion

The advent of NGS provides strong support for the differential diagnosis of benign/malignant of extrahepatic cholangiocarcinoma from cytological samples in cases of doubt. However, there are still a large number of non-contributory cases with a technic not always applicated in real clinical conditions, which justifies the search for new complementary tools. This study demonstrates that the proteomic profiling matching is a relevant approach able to correctly classify bile duct sample as benign or malignant while pathological examination gave indeterminate diagnosis. It is a new utilization of this technic which never has been reported before. The strength of the established proteomic signature was validated with the intern resampling and then validation with a new data set.

The majority of this diagnostic signature is composed of membrane surface proteins and extracellular matrix proteins, meaning that the stromal portion surrounding tumor cells is of relevant diagnostic value. In addition, analysis of the associated biological functions revealed a significant enrichment of proteins involved in cell adhesion, junction and epithelial-mesenchymal transition (EMT) processes which may have implication in cholangiocarcinoma's process. Indeed, we found in our signature the integrin aVb6 and integrin a5b1. Here, EMT constitutes a differential benign/malignant diagnostic element through the proteins involved with, which was not described before.

These results bring a proof-of-concept for a promising new tool in the field of the diagnosis of eCCA. Recently, different approaches using NGS panel on bile duct cytological samples of patient having bile duct stricture were developed with promising results. They have respectively shown that, compared with histological examination, NGS on bile duct samples improved sensitivity from 35% to 77% (Singhi et al. ([1])) in case of bile duct stricture and reached 72% when NGS was combined with histological results for detecting high grade dysplasia or CCA in patients with primary sclerosing cholangitis (Boyd et al. ([4])). It is relevant to noticed that NGS is a true innovation which clearly improves diagnosis for CCA. However, technical limitations exist with sufficient DNA amount required on bile duct samples. Furthermore, NGS principle is only to find mutations, which should have been already described and targeted with the panel kit. That's why proteomic profiling matching may have an additional place in the diagnosis part of eCCA. Indeed, proteomic analysis identifies a large number of proteins contained in a tissue (>2500), both from the few tumor cells present but also from its microenvironment. This may increase the probability of detecting proteins whose expression is associated with eCCA with improving the sensitivity. Our proof-of-concept with perfect matching of indeterminate cases is really encouraging and we can expect a very good sensitivity for subsequent analysis on a larger scale.

Interestingly, a transfer seems to be realizable as LC-MS/MS spectrometers becomes widespread in pathology services in expert center. The one-week turnaround time and reasonable cost are compatible with clinical requirements, since they are already routinely used to diagnose other pathologies.

In conclusion, the comparison of proteomic profiles provides a new tool for improving diagnostic for biliary stenosis. By giving a clear indication of a malignant diagnosis, as well as a benign one, it would reduce the number of necessary examinations, reduce preoperative uncertainty, and avoid unnecessary major surgeries.

### Reference List

1. Singhi AD, Nikiforova MN, Chennat J, Papachristou Gl, Khalid A, Rabinovitz M, et al. Integrating next-generation sequencing to endoscopic retrograde cholangiopancreatography (ERCP)-obtained biliary specimens improves the detection and management of patients with malignant bile duct strictures. Gut. 2020;69(1):52-61.
2. Dourthe C, Julien C, Di Tommaso S, Dupuy J, Dugot-Senant N, Brochard A, et al. Proteomic profiling of hepatocellular adenomas paves the way to new diagnostic and prognostic approaches. Hepatology. 2021; 74(3):1595-610.
3. Di Tommaso, Sylvaine et al. Spatial characterisation of β-catenin-mutated hepatocellular adenoma subtypes by proteomic profiling of the tumour rim. JHEP Reports, Volume 6, Issue 2, 100913.
4. Grubman SA, Perrone RD, Lee DW, Murray SL, Rogers LC, Wolkoff LI, et al. Regulation of intracellular pH by immortalized human intrahepatic biliary epithelial cell lines. Am J Physiol-gastr L. 1994;266(6):G1060-70.
5. Boyd S, Mustamäki T, Sjöblom N, Nordin A, Tenca A, Jokelainen K, et al. NGS of brush cytology samples improves the detection of high-grade dysplasia and cholangiocarcinoma in patients with primary sclerosing cholangitis: A retrospective and prospective study. Hepatol Commun. 2024;8(4):e0415.

## Claims

1. An *ex vivo* method for determining a reference proteomic profile of extrahepatic cholangiocarcinoma in the presence of bile duct stricture in a subject, comprising steps of :
a) quantification of the protein abundances of all proteins identified in at least one bile duct cytological sample from at least one subject for which a diagnosis of extrahepatic cholangiocarcinoma has been previously established, or having benign stricture,
b) quantification of the protein abundances of all proteins identified in human bile duct cells standard reference,
c) determining the proteomic profile of each of said at least one sample by comparing protein abundances of each protein in the at least one sample with respect to the standard reference,
d) determining a reference proteomic profile for extrahepatic cholangiocarcinoma condition, by means of a statistical test established with the proteomic profiles determined in step c).

2. Method according to claim 1, wherein said sample is obtainable by at least one technique selected among macrodissection, laser microdissection of a formalin-fixed paraffin-embedded or frozen tissue, fresh biopsy, and brushing.

3. Method according to claim 1 or 2, wherein the quantification of the protein abundances is realized by mass spectrometry.

4. Method according to any one of the preceding claims, wherein said at least one sample is at least five samples.

5. Method according to any one of the preceding claims, wherein said human bile duct cells standard reference is an immortalized normal human cholangiocytes cell line.

6. Method according to any one of the preceding claims, wherein comparing protein abundances of each protein in step c) is realized by calculating the ratio between protein abundances in the at least one sample and protein abundances in the standard reference.

7. Method according to any one of the preceding claims, wherein quantification is a label free quantification, a quantification with isotopic labelling, or a targeted quantification, preferably by mass spectrometry.

8. Reference proteomic profile of extrahepatic cholangiocarcinoma in the presence of bile duct stricture, obtained by the method as defined in any one of the preceding claims.

9. Reference proteomic profile according to claim 8, comprising extracellular proteins, cell adhesion proteins and epithelial-mesenchymal transition proteins.

10. Reference proteomic profile according to claim 8 or 9, wherein:
- said extracellular proteins comprise at least one protein selected in the group comprising COL15A1, MUC5B, TXNDC17, SRSF3, SNX1, RBM12 and FKBP1A protein,
- said cell adhesion proteins comprise at least one protein selected in the group comprising ITGAV, THBS1, UTRN, FN1, ITGA5, FERMT3, VASP, CFL1, ILK, TPM4, LRP1, CNN3, RPS14, FHL2 and PARK7 protein, and
- said epithelial-mesenchymal transition proteins comprise at least one protein selected in the group comprising ITGAV, THBS1, FN1, ITGA5, LRP1 and TPM protein.

11. An *ex vivo* method for determining proteomic profile of a bile duct cytological sample of a subject, comprising the following steps:
- quantification of the protein abundances of all proteins identified in said bile duct cytological sample,
- quantification of the protein abundances of all proteins identified in a human bile duct cells standard reference,
- determining the proteomic profile of said at least one sample by comparing protein abundances of each protein in the at least one sample with respect to the standard reference.

12. Method according to claim 11, wherein quantification is a label free quantification, a quantification with isotopic labelling or a targeted quantification.

13. An ex *vivo* method for differential diagnosis of extrahepatic cholangiocarcinoma in the presence of bile duct stricture of a subject, comprising the following steps:
- determining the proteomic profile of a bile duct cytological sample of the subject with a method as defined in claim 11,
- comparing the proteomic profile of said sample with a reference proteomic profile as defined in any one of claims 8 to 10,
Wherein:
- if the proteomic profile of said sample matches with a pathological reference proteomic profile, the subject is defined as having a extrahepatic cholangiocarcinoma,
- if the proteomic profile of said sample matches with a non-patholocial reference proteomic profile match, the subject is defined as having a benign lesion.
